# EUROPÄISCHE PATENTSCHRIFT

(11) **EP 1 328 344 B1**
(45) Veröffentlichungstag und Bekanntmachung des Hinweises auf die Patenterteilung: **15.08.2007**
(21) Anmeldenummer: 01982387.1
(22) Anmeldetag: 01.10.2001
(51) Int. Cl.: B01J 23/64, B01J 27/186, B01J 37/03, C23C 18/44, C23C 18/18, C01B 15/029, C07B 31/00

(54) **GETRÄGERTER, DURCH KONTROLLIERTE STROMLOSE ABSCHEIDUNG ERHÄLTLICHER PLATINMETALL-KATALYSATOR**
SUPPORTED CATALYST CONSISTING OF METAL OF THE PLATINUM GROUP AND OBTAINED BY MEANS OF CONTROLLED ELECTROLESS DEPOSITION
CATALYSEUR EN METAL DU GROUPE DU PLATINE SUR SUPPORT, OBTENU PAR DEPOT SANS COURANT CONTROLE

(30) Priorität: 02.10.2000 DE 10048844
(43) Veröffentlichungstag der Anmeldung: 23.07.2003
(73) Patentinhaber: BASF Aktiengesellschaft, 67056 Ludwigshafen (DE)
(72) Erfinder: BUTZ, Thomas, 67063 Ludwigshafen (DE); JUNICKE, Henrik, 68165 Mannheim (DE)
(74) Vertreter: Pohl, Michael Friedrich
(86) Internationale Anmeldenummer: PCT/EP2001/011346
(87) Internationale Veröffentlichungsnummer: WO 2002/028528

(56) Entgegenhaltungen:
- EP-A- 0 878 235
- DE-A- 2 046 521
- DE-A- 2 934 584
- US-A- 5 320 821

## Beschreibung

Die Erfindung betrifft einen geträgerten Platinmetall-Katalysator, ein Verfahren zu seiner Herstellung durch kontrollierte stromlose Abscheidung und ein Verfahren zur Hydrierung anorganischer und organischer Verbindungen unter Verwendung des Katalysators, insbesondere zur Direktsynthese von Wasserstoffperoxid.

Geträgerte Platinmetall-Katalysatoren werden in zahlreichen chemischen Prozessen, insbesondere zur Hydrierung anorganischer und organischer Verbindungen, eingesetzt und haben dort eine große technische Bedeutung erlangt. Eine intensiv untersuchte Umsetzung ist die so genannte Direktsynthese von Wasserstoffperoxid ausgehend von molekularem Sauerstoff und Wasserstoff. Man ist bestrebt, für diese Umsetzung und andere Hydrierungen Katalysatoren mit hoher Aktivität und Selektivität aufzufinden.

Die US 6,168,775 beschreibt Edelmetall-Katalysatoren für die Wasserstoffperoxid-Direktsynthese. Eine Edelmetallsalzlösung wird mit der Lösung eines ionischen Kontrollpolymers, z. B. eines Polyacrylats, mit einer Molmasse zwischen 300 und 8000 Dalton vermischt und vor oder nach Imprägnierung eines Trägers mit der Lösung mit Wasserstoff reduziert. Die so erhaltenen Katalysatoren sollen sich durch eine feine Verteilung des Edelmetalls auf dem Träger und durch bevorzugte Ausbildung von Kristallen mit 110-und/oder 220-Kristallflächen an der Oberfläche auszeichnen.

Viele Metalle können durch stromlose Abscheidung, auch autokatalytisches Abscheiden genannt, auf Träger aufgebracht werden. Die EP-A-0 878 235 beschreibt beispielsweise ein Verfahren zur Herstellung von geträgerten Katalysatoren, bei dem man einen porösen Träger mit einer Lösung eines Salzes des katalytisch aktiven Metalls und eines Reduktionsmittels behandelt und so eine stromlose Abscheidung des katalytisch aktiven Metalls erzielt.

Die WO 00/59635 beschreibt ein Verfahren zur Herstellung von Platinmetall-Katalysatoren auf metallischen Trägern, bei dem die Abscheidung des Metalls in Form diskreter Partikel erfolgt. Das Abscheiden erfolgt aus einem wässrigen Medium, in dem das Platinmetall komplexiert vorliegt und das einen pH-Wert von mehr als 4 aufweist.

Der Erfindung liegt die Aufgabe zugrunde, die Aktivität und/oder Selektivität der bekannten Platinmetall-Katalysatoren weiter zu verbessern, so dass die Raum-Zeit-Ausbeute der damit katalysierten Umsetzungen gesteigert werden kann. Das Platinmetall sollte darüber hinaus fest auf dem Träger verankert sein, damit auch unter mechanischer Belastung eine hohe Standzeit erzielt werden kann.

Erfindungsgemäß wird diese Aufgabe durch einen geträgerten Platinmetall-Katalysator gelöst, der durch kontrollierte stromlose Abscheidung wenigstens eines Platinmetalls aus einer Abscheidungslösung erhältlich ist, die
i) wenigstens eine homogen gelöste Platinmetallverbindung,
ii) ein Reduktionsmittel und
iii)wenigstens ein unter Isopolysäuren und Heteropolysäuren von Niob, Tantal, Molybdän, Wolfram und Vanadium oder deren Salzen ausgewähltes Kontrollagens enthält, wobei die dem Kontrollagens Zugrunde liegenden Element auf dem Katalysator nur in vernachlässigbarer Menge gefunden werden.

Die Erfindung betrifft außerdem ein Verfahren zur Hydrierung molekularer anorganischer oder organischer Verbindungen, bei dem man die zu hydrierende Verbindung in Gegenwart des erfindungsgemäßen Katalysators mit Wasserstoff in Kontakt bringt.

Vermutlich wird durch stromloses Abscheiden des Platinmetalls auf dem Träger in Gegenwart einer gelösten Isopolysäure und/oder Heteropolysäure eine homogenere Beschichtung, d. h. eine einheitlichere Verteilung der Edelmetall-Partikel und eine einheitlichere Partikelgröße auf dem Träger erreicht, die zu einer verbesserten Aktivität und/oder Selektivität führt. Die Homogenität der Beschichtung kann beispielsweise mit Hilfe der Raster-Elektronenmikroskopie und/oder Röntgen-Photoemissions-Spektroskopie (ESCA) untersucht werden.

Die Isopolysäure oder Heteropolysäure leitet sich von einem unter Niob, Tantal, Molybdän, Wolfram und Vanadium, vorzugsweise unter Molybdän, Wolfram und Vanadium ausgewähltem Element, besonders bevorzugt von Wolfram, ab. Unter Isopolysäuren werden anorganische Polysäuren verstanden, die partielle Anhydride der Orthosäuren dieser Elemente darstellen und nur Zentralatome einer Sorte enthalten. Beispiele dafür sind Heptamolybdänsäure, Hexawolframsäure, Dodecawolframsäure, Divanadiumsäure, Decavanadiumsäure, Hexaniobsäure, Hexatantalsäure. Unter Heteropolysäuren werden anorganische Polysäuren verstanden, die neben diesen Elementen ein weiteres Zentralatom, meist Arsen, Iod, Phosphor, Selen, Silicium oder Tellur, enthalten. Beispiele für solche Heteropolysäuren sind 12-Molybdophosphorsäure, 12-Wolframatophosporsäure, 12-Wolframatokieselsäure oder Hexawolframatoiodsäure. Die Iso- oder Heteropolysäure kann vorab gebildet und der Abscheidungslösung zugesetzt werden oder in situ in der Abscheidungslösung aus geeigneten Vorläuferverbindungen gebildet werden. Der Kondensationsgrad der Iso- und/oder Heteropolysäuren ist nicht kritisch. Der Kondensationsgrad, der sich beim pH-Wert der Abscheidungslösung einstellt, ist geeignet. Geeignete Vorläuferverbindungen sind monomere Oxosäuren, z. B. die Meso- oder Orthosäuren, bzw. oligomere Oxosäuren, z. B. Metasäuren der genannten Elemente oder deren Salze. Als Salze der Iso- und/oder Heteropolysäuren bzw. ihrer Vorläuferverbindungen kommen vor allem die Alkalimetallsalze, insbesondere die Natrium- und Kaliumsalze, oder die Ammoniumsalze in Betracht.

Geeignete Vorläuferverbindungen sind z. B. Natriumwolframat, Ammoniummetavanadat, Natriummolybdat und dergleichen.

Das molare Verhältnis von Platinmetall zu Iso- und/oder Heteropolysäure oder Vorläuferverbindung dafür in der Abscheidungslösung beträgt vorzugsweise 0,01 bis 5,0, insbesondere 0,1 bis 2,0, gerechnet als molares Verhältnis von Platinmetallatomen zu Niob-, Tantal-, Molybdän-, Wolfram- und/oder Vanadiumatomen.

Platinmetalle im Sinne der Erfindung sind die nicht zur Eisengruppe gehörenden Edelmetalle der 8. Nebengruppe des Periodensystems, nämlich Ruthenium, Rhodium, Iridium, Palladium, Osmium und Platin. Bevorzugt sind Ruthenium, Rhodium, Palladium und Platin, besonders bevorzugt sind Palladium und Platin. Geeignet sind auch Kombinationen der genannten Platinmetalle, bevorzugt sind Kombinationen aus Palladium und Platin, aus Palladium und Rhodium, aus Palladium und Iridium, aus Palladium, Platin und Rhodium und aus Palladium, Platin und Iridium. Besonders bevorzugt als Kombination ist Palladium und Platin. Bei den Kombinationen mit Palladium stellt Palladium vorzugsweise die Hauptkomponente dar. Der Palladiumanteil beträgt dann vorzugsweise mehr als 40 Gew.-%, vorzugsweise mehr als 60 Gew.-% und besonders bevorzugt mehr als 80 Gew.-%, bezogen auf den Gesamtgehalt an Platinmetall.

Die katalytisch aktive Komponente der erfindungsgemäßen Katalysatoren kann außer Platinmetallen noch weitere Elemente als Promotoren oder Dotierung enthalten, die die Aktivität und/oder Selektivität des Katalysators beeinflussen. Dazu zählen bevorzugt Metalle, wie Cobalt, Nickel, Kupfer, Silber, Gold, Chrom, Mangan, Rhenium, Aluminium, zinn, Blei, Arsen, Antimon und Wismut, und Nichtmetalle, wie Bor, Kohlenstoff, Silicium, Stickstoff und Phosphor.

Die als Promotoren oder Dotierungen eingesetzte Zusatzkomponenten machen in der Regel 0,01 bis 20 Gew.-%, vorzugsweise 0,1 bis 15 Gew.-% und insbesondere 0,5 bis 10 Gew.-%, bezogen auf den Platinmetallgehalt, aus.

Die Platinmetallverbindung ist unter Platinmetallsalzen und Platinmetallkomplexen ausgewählt. Bevorzugt werden Platinmetallkomplexe eingesetzt, insbesondere solche, in denen das Platinmetall in den Oxidationsstufen +1 bis +4 vorliegt. Vierfach koordinierte Komplexe sind bevorzugt. Insbesondere geeignet sind Palladium(II)komplexe, in denen Palladium in der Koordinationszahl 4 vorliegt.

Die Platinmetallkomplexe können unterschiedliche Liganden enthalten. Die Komplexe können vorab hergestellt oder in situ in der Abscheidungslösung gebildet werden. Geeignete negativ geladene Liganden sind z. B. ausgewählt unter Halogeniden und Pseudohalogeniden, wie Chlorid, Bromid, Jodid, CN, OCN und SCN, C₁-C₆-Carbonsäuren, wie Ameisensäure, Essigsäure und Propionsäure und deren Salzen, Chelatliganden, wie zum Beispiel Ethylendiamintetraessigsäure (EDTA), Nitrilotriessigsäure, 1,2-Diaminocyclohexantetraessigsäure und deren Salzen, Aminophosphonsäuren, wie Nitrilomethylenphosphonsäure, Diketonaten, wie Acetylacetonat, Hydroxycarbonsäuren, wie Glykolsäure, Milchsäure, Weinsäure und Gluconsäure, und deren Salzen. Geeignet als elektroneutrale Liganden sind z. B. Alkylnitrile, wie Acetonitril, Amine, wie Ammoniak, primäre, sekundäre und tertiäre C₁-C₆-Alkyl-Amine, wie Ethylamin, n-Propylamin, Isopropylamin, n-Butylamin, tert-Butylamin, Hexylamin, Dimethylamin, Diethylamin, Diisopropylamin, Di-n-butylamin, Trimethylamin, Triethylamin, Tripropylamin, N,N-Dimethylethylamin, N,N-Dimethylisopropylamin und N,N-Dimethylbutylamin, Di-, Tri-, Tetra- und Polyamine, wie Ethylendiamin, Diethylentriamin und Triethylentetramin, nichtaromatische und aromatische cyclische Amine, wie Pyrrolidin, Piperidin, Morpholin, Piperazin, Pyrrol und deren n-C₁-C₆-Alkylderivate, Pyridin und Phenanthrolin, Phosphine, wie tertiäre C₁-C₆-Alkyl- und C₆-C₁₂-Arylphosphine, insbesondere Triphenylphosphin, sowie Sulfide, wie C₁-C₆-Mono- und -Dialkylsulfide, C₆-C₁₂-Mono- und -Diarylsulfide und Sauerstoffverbindungen, Di-C₁-C₆-alkanole und Phenole sowie deren Ether.

Besonders bevorzugt sind stickstoffhaltige Liganden, insbesondere Amine, besonders bevorzugt Ammoniak. Vorzugsweise umfasst die Platinmetallverbindung einen Platinmetallkomplex mit einem stickstoffhaltigen Liganden.

Der Platinmetallgehalt der Abscheidungslösung liegt im Allgemeinen im Bereich von 0,001 bis 2 g/l, vorzugsweise im Bereich von 0,1 bis 0,5 g/l.

Bevorzugte Palladiumkomplexe sind H₂PdHal₄, M₂PdHal₄, M₂Pd(CN)₄, (NH₄)₂PdHal₄, Pd(NH₃)₄Hal₂, Pd(NH₃)₄(N0₃)₂ und Pd(NH₃)₄(CN)₂, wobei M für Alkalimetalle, insbesondere Natrium und Kalium, und Hal für Halogenatome, insbesondere für Chlor, Brom oder Iod, steht.

Bevorzugte weitere Platinmetallkomplexe sind (NH₄)₂IrCl₆, H₂PtC1₄, (NH₄)₂PtCl₄, Na₂PtCl₄ und K₂PtCl₄.

Darüber hinaus enthält die Abscheidungslösung wenigstens ein Reduktionsmittel in gelöster Form. Geeignet als Reduktionsmittel sind alle Stoffe oder Stoffgemische, deren Redoxpotential unterhalb des Redoxpotentials der eingesetzten Platinmetallverbindung liegt. Bevorzugt sind Stoffe mit einem Standardpotential in wässrigem Medium von weniger als +0,5 Volt, vorzugsweise mit einem Standardpotential von weniger als 0 Volt.

Beispiele für geeignete Reduktionsmittel sind Ameisensäure oder α-Hydroxycarbonsäuren, wie Citronensäure, Milchsäure, Weinsäure und insbesondere die Salze der Carbonsäuren, bevorzugt die Alkali-, Erdalkali-, Ammonium- und C₁-C₁₀-Alkylammoniumsalze, phosphorige oder hypophosphorige Säure, die Salze der phosphorigen oder hypophosphorigen Säure, insbesondere die Alkalimetall- oder Erdalkalimetallsalze, C₁-C₁₀-Alkanole, wie Methanol, Ethanol und Isopropanol, Zucker, wie Aldosen und Ketosen in Form von Mono-, Di- und Oligosacchariden, insbesondere Glucose, Fructose und Lactose, Aldehyde, wie Formaldehyd, Borwasserstoffverbindungen, wie z. B. Borhydride, Borane, Metallboranate und Borankomplexe, z. B. Diboran, Natriumborhydrid und Aminoborane, insbesondere Trimethylaminboran, Hydrazin und Alkylhydrazine, wie Methylhydrazin, Hydrogendithionite und Dithionite, insbesondere Natrium- und Kaliumhydrogendithionit, Natrium-, Kalium- und Zinkdithionit, Hydrogensulfite und Sulfite, insbesondere Natrium- und Kaliumhydrogensulfit, Natrium-, Kalium- und Calciumsulfit, Hydroxylamin und Harnstoff, sowie Gemische davon.

Bevorzugte Reduktionsmittel sind Natrium- und Kaliumhypophosphit, Ammoniumformiat, Trimethylamin-boran, Natriumborhydrid, Natriumdithionit und Natriumhydrogendithionit, sowie Gemische von Ammoniumformiat und Natriumhypophosphit.

In der Regel wird mindestens ein Redoxäquivalent, bezogen auf die Summe der Platinmetalle und Zusatzkomponenten (z. B. Promotoren/Dotierungskomponenten), an Reduktionsmittel eingesetzt. Bevorzugt wird das Reduktionsmittel im Überschuss eingesetzt. Insbesondere geeignet ist ein molares Verhältnis von Reduktionsmittel zu Platinmetall von 10:1 bis 100:1 und besonders bevorzugt 20:1 bis 60:1, wie zum Beispiel etwa 30:1, etwa 40:1 oder etwa 50:1.

Bevorzugt weist die Abscheidungslösung einen pH-Wert von mehr als 6 auf. Dieser liegt vorzugsweise in einem Bereich von 7 bis 14, insbesondere 8 bis 12. Dazu kann es erforderlich sein, zu der Abscheidungslösung eine Base zuzugeben, um den gewünschten pH-Wert zu erreichen. Basen sind alle Stoffe bzw. Verbindungen, die geeignet sind, den pH-Wert des wässrigen Mediums auf den gewünschten Wert einzustellen. Insbesondere werden solche Basen eingesetzt, die komplexstabilisierende Eigenschaften aufweisen, d. h. zumindest partiell Lewis-Basencharakter aufweisen. Vorzugsweise wird die Base ausgewählt unter Metalloxiden, Metallhydroxiden, insbesondere Alkalimetallhydroxiden, wie Natriumhydroxid und Kaliumhydroxid, Metallcarbonaten, insbesondere Alkalimetall- und Erdalkalimetallcarbonaten, wie Lithiumcarbonat, Natriumcarbonat, Kaliumcarbonat, Magnesiumcarbonat und Calciumcarbonat, Stickstoffbasen, insbesondere Ammoniak, primäre, sekundäre und tertiäre Aminen, wie die zuvor bei den stickstoffhaltigen Komplexliganden beschriebenen. Ebenfalls geeignet sind Puffersysteme, insbesondere solche aus den vorgenannten Basen, den Salzen der vorgenannten Basen und/oder geeigneten Säuren. Besonders bevorzugte Basen sind Ammoniak und Natronlauge.

Die Abscheidungslösung ist in der Regel wässrig, d. h. sie enthält mindestens 10 Gew.-%, vorzugsweise mindestens 30 Gew.-% und insbesondere mindestens 50 Gew.-% Wasser. Der von Wasser verschiedene Teil ist vorzugsweise ausgewählt unter mit Wasser mischbaren Lösungsmitteln, wie Methanol, Ethanol, n-Propanol, Isopropanol, n-Butanol, sec-Butanol, tert-Butanol, Tetrahydrofuran, Dioxan.

Als Träger können alle dem Fachmann bekannten Katalysatorträger verwendet werden, sowohl metallische als auch nicht-metallische. Geeignete metallische Träger sind beispielsweise beschrieben in WO 00/59635, auf deren Offenbarung verwiesen wird. Nichtmetallische Träger sind in der Regel ausgewählt unter mineralischen Werkstoffen oder Kunststoffen.

Der Ausdruck "mineralischer Werkstoff" umfasst vorliegend ganz allgemein nichtmetallische anorganische Werkstoffe, wie natürliche und synthetische Mineralien, Gläser, Keramiken etc. Vorzugsweise wird als mineralischer Werkstoff ein Glas eingesetzt. Bevorzugt sind Gläser aus geschmolzenem Siliciumdioxid oder geschmolzenem Quarz sowie Gläser auf Basis von Alkali-, Erdalkali-, Boro-, Alumino- und Bleisilicat. Bevorzugte mineralische Trägermaterialien sind weiterhin auch Borat-, Phosphat-, Germanat-, Chalkogenid- und Halogenidgläser, wie z. B. aus Berylliumfluorid.

Vorzugsweise ist der als Träger eingesetzte mineralische Werkstoff weiterhin ausgewählt aus keramischen Materialien. Geeignete keramische Materialien können aus Metalloxiden, -boriden, -nitriden und/oder -carbiden hergestellt werden. Die erfindungsgemäß eingesetzten keramischen Materialien können glasiert oder unglasiert, kristallin oder teilkristallin sein. Bevorzugt werden für das erfindungsgemäße Verfahren Keramiken aus Basismaterialien eingesetzt, die ausgewählt sind unter Aluminiumoxid, Siliciumcarbid, Siliciumnitrid, Zirkondioxid und Mischungen davon. Vorzugsweise werden weiterhin Keramiken eingesetzt, die Kationen enthalten, wie das z. B. in Chelatit, Steatit, Cordierit, Anorthit, Mullit oder Pollucit der Fall ist. Bevorzugt sind weiterhin keramische Kompositmaterialien.

Nicht-metallische Träger müssen in der Regel aktiviert werden. Unter "*Aktivierung*" des Trägers wird ein Vorgang verstanden, bei dem auf der Oberfläche des Trägers Keime ausgebildet werden, die die anschließende stromlose Abscheidung des Platinmetalls fördern. Die Keime bestehen in der Regel aus einem Metall, vorzugsweise einem Platinmetall, insbesondere Palladium. Zur Aktivierung wird der Träger vorzugsweise mit der Lösung eines Sensibilisators behandelt und anschließend mit der Lösung eines Platinmetallsalzes behandelt. Als Sensibilisator sind allgemein Reduktionsmittel geeignet, wobei Zinn(II)-Verbindungen, insbesondere Zinn(II)-chlorid, und Titan(III)-verbindungen besonders bevorzugt sind, gegebenenfalls in Kombination mit anderen Reduktionsmitteln. Weitere als Sensibilisatoren vorteilhafte Reduktionsmittel sind Salze der Hypophosphorigen Säure. Die Aktivierung ist beispielsweise in der EP-A-0 878 235 beschrieben, auf deren Offenbarung verwiesen wird.

Die Abscheidung erfolgt im Allgemeinen bei einer Temperatur im Bereich von 0 bis 100 °C, vorzugsweise im Bereich von 30 bis 100 °C und insbesondere im Bereich von 40 bis 85 °C.

Beispielsweise wird der Träger zur Abscheidung des Platinmetalls mit der vollständig formulierten Abscheidungslösung in Kontakt gebracht. Alternativ kann der Träger zunächst mit einer Lösung des Reduktionsmittels und des Kontrollagens, die gegebenenfalls die fakultativen Bestandteile der Abscheidungslösung ganz oder teilweise enthält, in Kontakt gebracht werden. Das Platinmetall und die übrigen fakultativen Bestandteile werden dann bei der Abscheidungstemperatur oder einer z. B. bis zu 30 °C tieferen Temperatur zugesetzt. Weiter alternativ kann man den Träger mit einer Lösung der Platinmetallverbindung und des Kontrollagens in Kontakt bringen und dann eine Lösung des Reduktionsmittels hinzufügen.

Im erfindungsgemäßen Verfahren hat es sich als vorteilhaft erwiesen, während der Abscheidung des Platinmetalls auf dem Träger für eine ausreichende Umwälzung der Abscheidungslösung zu sorgen, z. B. durch Pumpen oder Rühren.

Die Abscheidungslösung wird in ausreichend längerem Kontakt mit dem Träger belassen, um die stromlose Abscheidung des Platinmetalls auf dem Träger zu erreichen. Die erforderliche Reaktionszeit liegt in der Regel zwischen 0,5 und 500 Minuten, vorzugsweise 1 und 300 Minuten und besonders bevorzugt zwischen 2 und 60 Minuten. In der Regel werden mehr als 70 Gew.-%, vorzugsweise mehr als 80 Gew.-% und besonders bevorzugt mehr als 90 Gew.-% der eingesetzten Platinmetalle auf dem Träger abgeschieden. Dabei wird das Platinmetall in der Regel so fest an den Träger gebunden, dass es beim Einsatz in katalytischen Reaktionen durch den Kontakt mit Flüssigkeiten und Gasen nicht nennenswert abgelöst wird. Die dem Kontrollagens zugrunde liegenden Elemente werden auf dem Katalysator nur in vernachlässigbarer Menge gefunden.

Die erfindungsgemäßen Katalysatoren eignen sich zur Hydrierung von Sauerstoff zur Herstellung von Wasserstoffperoxid.

Ein weiterer Gegenstand der vorliegenden Erfindung ist ein Verfahren zur Herstellung von Wasserstoffperoxid, wobei man den Sauerstoff und den Wasserstoff in einem flüssigen Medium, vorzugsweise einer im Wesentlichen wässriger Lösung mit einem erfindungsgemäßen Katalysator in Kontakt bringt.

Geeignete Reaktoren für die Synthese von H₂O₂ sind beispielsweise in den EP-A-068 862, EP-A-201 614 und der EP-A-448 884 beschrieben. Besonders bevorzugt sind Rohrreaktoren, in denen der erfindungsgemäße Katalysator als Schüttung vorliegt oder in Form von zylindrisch aufgebauten Katalysatoreinheiten eingepasst ist. Durch entsprechende Formgebung für die Träger, wie zuvor beschrieben, kann für optimale Strömungsverhältnisse für Gas und Flüssigkeit gesorgt werden.

Bevorzugt rieselt die flüssige Phase von oben nach unten über die Katalysatorschüttung. Dabei kann das Gas im Gleichstrom oder im Gegenstrom geführt werden kann, bevorzugt im Gleichstrom.

Bevorzugt kann der Wasserstoff dem Reaktor über eine oder mehrere Zwischeneinspeisungen stromabwärts vom Einspeisungspunkt des Sauerstoffs oder der Luft zugeführt werden. Die Leerrohrgeschwindigkeit von Reaktionsgas und Reaktionsmedium liegt vorzugsweise in einem Bereich von etwa 20 bis 7000 m/h, besonders bevorzugt in einem Bereich von 50 bis 1400 m/h.

Als Reaktionsmedium dient vorzugsweise Wasser und/oder C₁-C₃-Alkanole, insbesondere Wasser und/oder Methanol. Wenn als Reaktionsmedium Wasser verwendet wird, kann diesem bis zu 20 Gew.-% des Alkohols, vorzugsweise Methanol, zugesetzt werden. Wird ein alkoholisches Reaktionsmedium eingesetzt, kann dieses zu 40 Gew.-%, vorzugsweise bis zu 20 Gew.-% und besonders bevorzugt bis zu 5 Gew.-% Wasser enthalten. Ganz besonders bevorzugt wird Wasser als alleiniges Reaktionsmedium verwendet. Zur Stabilisierung des Wasserstoffperoxids gegen Zersetzung werden dem Reaktionsmedium Säuren, deren pKa-Wert vorzugsweise kleiner als der der Essigsäure ist, insbesondere Mineralsäuren, wie Schwefelsäure, Phosphorsäure oder Salzsäure, zugesetzt. Die Säurekonzentration beträgt in der Regel wenigstens 10⁻⁴ Mol/Liter, vorzugsweise 10⁻³ bis 10⁻¹ Mol/Liter. Weiterhin werden in der Regel noch Spuren von Bromid oder Chlorid in Konzentrationen von 1 bis 1000 ppm, vorzugsweise 5 bis 700 ppm und besonders bevorzugt 50 bis 600 ppm zugesetzt. Es können aber auch andere Stabilisatoren, wie z. B. Formaldehyd, verwendet werden.

Das Reaktionsgas, das neben Wasserstoff und Sauerstoff auch noch inerte Gase wie Stickstoff oder Edelgase enthalten kann, weist in der Regel O₂:H₂-Verhältnisse im Bereich von 2:1 bis 1000:1 auf. Vorzugsweise werden Molverhältnisse im Bereich von 5:1 bis 100:1, insbesondere 20:1 bis 100:1 eingesetzt. Der im Reaktionsgas verwendete Sauerstoff kann auch in Form von Luft dem Reaktionsgas zugemischt werden.

In einer bevorzugten Ausführungsform wird das Reaktionsgas im Kreis geführt. In diesem Fall liegt das Molverhältnis im Frischgasgemisch in der Nähe der Stöchiometrie, vorzugsweise im Bereich von 1,5:1 bis 0,5:1. Das Molverhältnis O₂:H₂ im Kreisgas sollte im Bereich von 5:1 bis 1000:1, vorzugsweise im Bereich von 20:1 bis 100:1 liegen. Die Reaktion kann bei Normaldruck als auch bei Überdrucken bis zu 200 bar durchgeführt werden. Vorzugsweise beträgt der Druck 10 bis 100 bar, insbesondere 10 bis 80 bar. Die Reaktionstemperatur kann im Bereich von 0 bis 80 °C liegen, vorzugsweise wird im Bereich von 5 bis 60 °C und insbesondere von 25 bis 55 °C gearbeitet. Vorzugsweise werden die Partialdrücke der Reaktionsgase in der Reaktionsgasmischung im Reaktor als auch im Kreisgas so gewählt, dass sich unter Reaktionsbedingungen die Wasserstoffkonzentration unterhalb der unteren Explosionsgrenze befindet.

Durch das beschriebene Verfahren lassen sich Wasserstoffperoxidlösungen mit Wasserstoffgehalten oberhalb 2 Gew.-%, vorzugsweise im Bereich von 3 bis 25 Gew.-% herstellen. Die Konzentration kann durch Einstellung der Stoffströme in der gewünschten Weise vorgewählt werden. Langzeituntersuchungen haben gezeigt, dass auch nach mehr als 40 Tagen Betriebsdauer keine oder nur eine geringfügige Abnahme der Katalysatoraktivität und Selektivität zu verzeichnen ist.

Im Gegensatz zu aus dem Stand der Technik bekannten Katalysatoren besitzen die erfindungsgemäß hergestellten Katalysatoren ausgezeichnete Katalysatoreigenschaften. Sie sind in Hydrierreaktionen hochaktiv und sehr selektiv. Außerdem wird durch das Verfahren eine besonders gute Haftung der Platinmetall-Partikel auf dem Träger erzielt, weshalb mit den erfindungsgemäß hergestellten Katalysatoren besonders hohe Standzeiten erreichen.

Die Erfindung wird anhand der beigefügten Figur und der nachfolgenden Beispiele näher veranschaulicht.

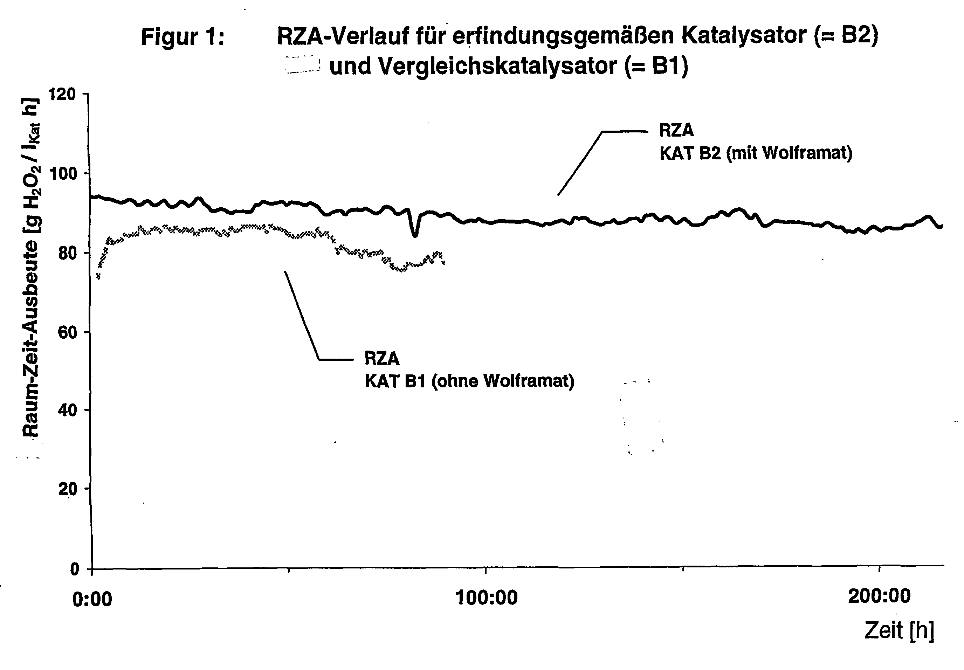

Fig.1 zeigt den zeitlichen Verlauf der Raum-Zeit-Ausbeute für die durch einen erfindungsgemäßen Katalysator bzw. einen Vergleichskatalysator katalysierte Wasserstoffperoxid-Direktsynthese.

### Herstellung der Katalysatoren

### Beispiel 1 (= Vergleichsbeispiel, Kat. B1):

1200 g Steatit-Träger der Fa. Ceramtec in Form von Kugeln mit 2 mm Durchmesser wurden nacheinander mit Natronlauge, 25%-iger Schwefelsäure und dest. Wasser gewaschen. Die so vorbehandelten Kugeln wurden anschließend auf einer Nutsche aktiviert, indem man 3 min eine Lösung A (= 5 g/l SnCl₂ + 10 ml/l konz. Salzsäure) einwirken ließ, dann nach Abfiltrieren und Waschen mit 0,5 l dest. Wasser 3 min eine Lösung B (= 0,2 g/l PdCl₂ + 1ml/l konz. Salzsäure) einwirken ließ. Nach erneutem Abfiltrieren und Waschen mit Wasser wurde die gesamte Prozedur wiederholt und zuletzt dreimal mit je 0,5 1 dest. Wasser gespült.

Die Kugeln wurden feucht in ein Glasrohr eingebaut und mit einer Lösung C versetzt (= 70 g/l NH₄Cl, 23 g/l NH₃, 30 g/l Natriumhypophosphit). Diese Lösung wurde mit Hilfe einer Pumpe so durch das Rohr gepumpt, dass die Kugeln sich gerade bewegen und ein Fließbett bildeten. Über ein Reservoir, das gleichzeitig als Gasabscheider dient, wurde die Lösung wieder an den Eingang des Glasrohres zurückgeführt. Das Glasrohr war außerdem mit einem Doppelmantel versehen, der mit Heizflüssigkeit gefüllt ist. Die Temperatur in der Lösung wurde vor der Beschichtung unter Umpumpen auf 40 +/- 0,5 °C gebracht. Dann wurden 20 g einer Lösung D von 2,8 mg Hexachloroplatinsäure und 526 mg Natrium-tetra-chloro-palladat in Wasser zugegeben und die Reaktion gestartet. Nach 1 Stunde war die Reaktion beendet. Man ließ die Lösung ablaufen und spülte mit Wasser nach. Der so hergestellte Katalysator hatte einen Pd-Gehalt von 145 mg/kg, entsprechend einem Abscheidegrad von 92 %.

### Beispiel 2 (= Kat. B2):

Die Katalysatorpräparation aus Beispiel 1 wurde wiederholt mit dem Unterschied, dass die Lösung D zusätzlich 405 mg Natriumwolframat enthielt.

Die Eigenschaften der Katalysatoren wurden in der Direktsynthese von Wasserstoffperoxid aus Wasserstoff und Sauerstoff überprüft.

### Beispiel 3:

Ein Doppelmantelreaktor mit einem Innendurchmesser von 2,1 cm und einer Länge von 2,00 m wurde mit dem Katalysator B1 beschickt. Bei 40 °C und 50 bar Druck ließ man eine Lösung von 5 g/l Phosphorsäure und 120 mg/l Bromwasserstoff in Wasser mit einer Geschwindigkeit von 1,0 kg/h über das Katalysatorbett rieseln. Gleichzeitig wurde mit Hilfe eines Gaskompressors ein Gemisch von 3 % Wasserstoff und 97 % Sauerstoff mit einer Geschwindigkeit von 10 400 Nl/Stunde von oben nach unten im Kreis über das Katalysatorbett gepumpt. Das Gasgemisch wird mit Hilfe von zwei Massendurchflussmessern für Wasserstoff und Sauerstoff erzeugt. Seine Zusammensetzung wird mit Hilfe eines Wärmeleitdetektors, über den ein kleiner Teilstrom als Abgasstrom geleitet wurde, ermittelt und nachgeregelt.

Die Menge an durch die Reaktion zu Wasserstoffperoxid und Wasser verbrauchten Wasserstoffs wurde aus den eingeleiteten Massenströmen der Gase und aus dem Abgasstrom errechnet.

Das aus dem Reaktionsrohr austretende Produktgemisch wurde in einem Abscheider noch unter Druck von den Gasen getrennt und flüssig aus der Anlage herausgefördert. Der Massenstrom wurde gegen den Zulaufstrom bilanziert. Der Wasserstoffperoxidgehalt in dem flüssigen Austrag wurde durch Titration bestimmt.

Aus der Masse des Austragsstroms, dem Gehalt an Wasserstoffperoxid und der Menge verbrauchten Wasserstoffs wurde die Selektivität bezogen auf Wasserstoff errechnet. Der Raum-Zeit-Umsatz (RZU) ergibt sich als Verbrauch von Wasserstoff in mol pro Zeiteinheit bezogen auf das Volumen der Katalysatorschüttung.

Die Raum-Zeit-Ausbeute (RZA) ergibt sich aus der pro Zeiteinheit gebildeten Menge Wasserstoffperoxids bezogen auf das Volumen von 690 ml Katalysatorschüttung in dem Rohrreaktor. Die Ergebnisse von sind in der nachfolgenden Tabelle 1 zusammengestellt. Den zeitlichen Verlauf der Raum-Zeit-Ausbeute zeigt Fig. 1.

### Beispiel 4:

Beispiel 3 wurde wiederholt mit dem Unterschied, dass der Katalysator B2 verwendet wurde. Diese. Maßnahme führt zu einer höheren Raum-Zeit-Ausbeute aufgrund einer höheren Aktivität und einer etwas besseren Selektivität (vgl. Tabelle 1 und Fig. 1). Man sieht, dass die RZA für den erfindungsgemäl3en Katalysator stets oberhalb des RZA des Vergleichskatalysators verläuft.

| Nr. | Katalysator | T [°C] | Selektivität | RZU [molH₂/lh] | RZA [gH₂O₂/lh] | Laufzeit |
|---|---|---|---|---|---|---|
| 3 | B1 | 50 °C | 79 % | 3,0-2,7 | 85-77 | 90 h |
| 4 | B2 | 50 °C | 81 % | 3,4-3,0 | 100-85 | >400 h |

## Patentansprüche

1. Geträgerter Platinmetall-Katalysator, erhältlich durch kontrollierte stromlose Abscheidung wenigstens eines Platinmetalls aus einer Abscheidungslösung, die
i) wenigstens eine homogen gelöste Platinmetallverbindung,
ii) ein Reduktionsmittel und
iii)wenigstens ein unter Isopolysäuren und Heteropolysäuren von Niob, Tantal, Molybdän, Wolfram und Vanadium oder deren Salze ausgewähltes Kontrollagens enthält, wobei die dem Kontrollagens zugrunde liegenden Elemente auf dem Katalysator nur in vernachlässigbarer Menge gefunden werden.

2. Katalysator nach Anspruch 1, wobei das Platinmetall unter Palladium oder einer Kombination von Palladium und Platin ausgewählt ist.

3. Katalysator nach Anspruch 1 oder 2, wobei die Platinmetallverbindung einen Platinmetallkomplex mit einem stickstoffhaltigen Liganden umfasst.

4. Katalysator nach einen der vorhergehenden Ansprüche, wobei die Abscheidungslösung einen pH-Wert von 8 bis 12 aufweist.

5. Katalysator nach einem der vorhergehenden Ansprüche, wobei die Isopolysäure oder Heteropolysäure in situ in der Abscheidungslösung aus einer monomeren oder oligomeren Oxosäure gebildet ist.

6. Katalysator nach einem der vorhergehenden Ansprüche, wobei der Träger vor der Abscheidung aktiviert worden ist.

7. Katalysator nach Anspruch 6, wobei der Träger zur Aktivierung mit einer Lösung eines Sensibilisators behandelt und anschließend mit der Lösung eines Platinmetallsalzes behandelt worden ist.

8. Katalysator nach einem der vorhergehenden Ansprüche, wobei der Träger ausgewählt ist unter keramischen Werkstoffen und Gläsern.

9. Verfahren zur Herstellung von Wasserstoffperoxid durch Hydrierung molekularen Sauerstoffs, bei dem man der molekulare Sauerstoff in Gegenwart eines Katalysators nach einem der Ansprüche 1 bis 8 mit Wasserstoff in Kontakt bringt.

10. Verfahren nach Anspruch 9, wobei man den Sauerstoff und den Wasserstoff in einem flüssigen Medium mit dem Katalysator in Kontakt bringt.

11. Verfahren zur Herstellung eines Katalysators nach einem der Ansprüche 1 bis 8, bei dem man wenigstens ein Platinmetall auf einem Träger aus einer Abscheidungslösung stromlos abscheidet, die
i) wenigstens eine homogen gelöste Platinmetallverbindung,
ii) ein Reduktionsmittel und
iii) wenigstens ein unter Isopolysäuren und Heteropolysäuren von Niob, Tantal, Molybdän, Wolfram und Vanadium oder deren Salze ausgewähltes Kontrollagens enthält.

## Claims

1. A supported platinum group metal catalyst obtainable by controlled electroless deposition of at least one platinum group metal from a deposition solution which comprises
i) at least one homogeneously dissolved platinum group metal compound,
ii) a reducing agent and
iii) at least one control agent selected from isopolyacids and heteropolyacids of niobium, tantalum, molybdenum, tungsten and vanadium or their salts, the elements on which the control agent is based being detected on the catalyst only in negligible amounts.

2. The catalyst according to claim 1, wherein the platinum group metal is selected from palladium or a combination of palladium and platinum.

3. The catalyst according to claim 1 or 2, wherein the platinum group metal compound comprises a platinum group metal complex having a nitrogen-containing ligand.

4. The catalyst according to any of the preceding claims, wherein the deposition solution has a pH of from 8 to 12.

5. The catalyst according to any of the preceding claims, wherein the isopolyacid or heteropolyacid is formed in situ in the deposition solution from a monomeric or oligomeric oxoacid.

6. The catalyst according to any of the preceding claims, wherein the support has been activated before the deposition.

7. The catalyst according to claim 6, wherein the support has been treated to activate it with a solution of a sensitizer and then with the solution of a platinum group metal salt.

8. The catalyst according to any of the preceding claims, wherein the support is selected from ceramic materials and glasses.

9. A process for preparing hydrogen peroxide by hydrogenating molecular oxygen, in which the molecular oxygen is contacted with hydrogen in the presence of a catalyst according to any of claims 1 to 8.

10. The process according to claim 9, wherein the oxygen and the hydrogen are contacted with the catalyst in a liquid medium.

11. A process for preparing a catalyst according to any of claims 1 to 8, in which at least one platinum group metal is deposited onto a support by electroless deposition from a deposition solution which comprises
i) at least one homogeneously dissolved platinum group metal compound,
ii) a reducing agent and
iii)at least one control agent selected from isopolyacids and heteropolyacids of niobium, tantalum, molybdenum, tungsten and vanadium or their salts.

## Revendications

1. Catalyseur en métal du groupe du platine sur support, que l'on peut obtenir par déposition contrôlée sans courant d'au moins un métal du groupe du platine d'une solution de déposition, qui contient
i) au moins un composé de métal du groupe du platine dissous de manière homogène,
ii)un réducteur, et
iii) au moins un agent de contrôle choisi parmi des isopolyacides et hétéropolyacides de niobium, de tantale, de molybdène, de tungstène et de vanadium ou leurs sels, les éléments à la base de l'agent de contrôle n'étant trouvés sur le catalyseur qu'en quantité négligeable.

2. Catalyseur suivant la revendication 1, dans lequel le métal du groupe du platine est choisi parmi du palladium ou une combinaison de palladium et de platine.

3. Catalyseur suivant la revendication 1 ou 2, dans lequel le composé de métal du groupe du platine comporte un complexe de métal du groupe du platine avec un ligand azoté.

4. Catalyseur suivant l'une des revendications précédentes, dans lequel la solution de déposition présente une valeur de pH de 8 à 12.

5. Catalyseur suivant l'une des revendications précédentes, dans lequel l'isopolyacide ou hétéropolyacide est formé in situ dans la solution de déposition à partir d'un oxacide monomère ou oligomère.

6. Catalyseur suivant l'une des revendications précédentes, dans lequel le support a été activé avant la déposition.

7. Catalyseur suivant la revendication 6, dans lequel le support a été, pour l'activation, traité avec une solution d'un agent sensibilisant et a ensuite été traité avec la solution d'un sel de métal du groupe du platine.

8. Catalyseur suivant l'une des revendications précédentes, dans lequel le support est choisi parmi des matériaux céramiques et des verres.

9. Procédé de préparation de peroxyde d'hydrogène par hydrogénation d'oxygène moléculaire, dans lequel on met en contact l'oxygène moléculaire avec l'hydrogène en présence d'un catalyseur suivant l'une des revendications 1 à 8.

10. Procédé suivant la revendication 9, dans lequel on met l'oxygène et l'hydrogène en contact avec le catalyseur dans un milieu liquide.

11. Procédé de préparation d'un catalyseur suivant l'une des revendications 1 à 8, dans lequel on dépose sans courant au moins un métal du groupe du platine sur un support d'une solution de séparation qui contient
i) au moins un composé de métal du groupe du platine dissous de manière homogène,
ii)un réducteur, et
iii) au moins un agent de contrôle choisi parmi des isopolyacides et hétéropolyacides de niobium, de tantale, de molybdène, de tungstène et de vanadium ou leurs sels.
